# FASCICULE DE BREVET EUROPEEN

(11) **EP 1 459 139 B1**
(45) Date de publication et mention de la délivrance du brevet: **06.09.2006**
(21) Numéro de dépôt: 02801100.5
(22) Date de dépôt: 04.12.2002
(51) Int. Cl.: G05D 21/02

(54) **PROCEDE POUR GARANTIR AU MOINS UNE CARACTERISTIQUE D'UN FLUIDE UTILISE POUR LA PRODUCTION DE PRODUITS ALIMENTAIRES**
VERFAHREN ZUR SICHERSTELLUNG MINDESTENS EINER EIGENSCHAFT EINER FLÜSSIGKEIT, DIE BEIM HERSTELLEN VON NAHRUNGSMITTELN VERWENDET WIRD
METHOD FOR GUARANTEEING AT LEAST ONE CHARACTERISTIC OF A FLUID USED FOR PRODUCING FOOD PRODUCTS

(30) Priorité: 06.12.2001 FR 0115787
(43) Date de publication de la demande: 22.09.2004
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: VIE, Jean-François, 78000 Versailles (FR); LEDON, Henry, 78000 Versailles (FR); GIRARDON, Philippe, 78000 Versailles (FR); GIRAULT, Christel, 78960 Voisins le Bretonneux (FR)
(74) Mandataire: Mellul-Bendelac, Sylvie Lisette
(86) Numéro de dépôt international: PCT/FR2002/004174
(87) Numéro de publication internationale: WO 2003/048875

(56) Documents cités:
- DE-A- 19 918 899
- FR-A- 1 176 831
- FR-A- 1 466 278
- FR-A- 2 735 381
- US-B1- 6 232 204

## Description

La présente invention concerne un procédé pour garantir au moins une caractéristique d'un fluide utilisé pour la production de produits alimentaires.

Les exigences en matière de sécurité et de qualité sont de plus en plus importantes dans l'industrie alimentaire.

Ainsi, il apparaît souhaitable de garantir que certaines caractéristiques des fluides utilisés pour la production de produits alimentaires possèdent des valeurs acceptables. Ces caractéristiques sont par exemple des caractéristiques relatives à la teneur en impuretés chimiques, physiques ou biologiques.

Les fluides auxquels on se réfère peuvent être utilisés en tant qu'auxiliaire technologique et ne se retrouvent donc pas dans ou au contact des produits alimentaires finis au moment de leur consommation. Il s'agit par exemple des fluides cryogéniques utilisés pour refroidir les produits alimentaires.

De tels fluides peuvent également être utilisés en tant qu'additifs ou qu'ingrédients et restent donc dans ou au contact des produits alimentaires finis. Il s'agit par exemple des fluides utilisés en tant que propulseurs ou pour former des atmosphères protectrices ou pour modifier le pH.

On connaît de EP-932 007 un procédé de filtration en phase liquide d'un fluide cryogénique pour enlever des micro-organismes et/ou des particules physiques. Le fluide cryogénique est par exemple utilisé dans l'industrie agroalimentaire. Ce procédé de filtration ne comprend pas d'étape de mesure d'une caractéristique du fluide cryogénique.

US-4 759 848 décrit un procédé de stérilisation par filtration d'un liquide cryogénique. Le procédé ne comprend pas non plus d'étape de mesure d'une caractéristique du liquide cryogénique.

FR-2 728 803 décrit un procédé de fourniture d'air sec comprenant des étapes de purification d'air comprimé mais aucune étape de mesure d'une caractéristique de cet air.

WO-98/48 259 décrit un procédé de différenciation quantitative et qualitative en ligne des particules biotiques et abiotiques d'un gaz. Ce procédé peut être utilisé dans le domaine alimentaire.

US-5 428 555 décrit un système d'obtention et d'analyse d'informations relatives à un procédé de production de pastilles semi-conductrices utilisant un gaz. Ce document ne mentionne pas d'étape de mesure d'une caractéristique du gaz.

EP-584 747 décrit l'utilisation d'hélium de haute pureté pour la production de produits. Une mesure de pureté est effectuée en aval de dispositifs de purification raccordés en parallèle et contenant des agents de dessiccation, d'adsorption et/ou des catalyseurs d'oxydation. Une telle mesure permet de déterminer si l'hélium doit passer par l'un ou l'autre ou par les deux dispositifs pour assurer une purification satisfaisante. L'hélium n'est pas un fluide utilisé pour la production de produits alimentaires, il peut être utilisé comme traceur d'éventuelles fuites au niveau d'un emballage. Le procédé décrit n'est en outre pas adapté à une telle production car il ne permet pas de garantir que la production des produits a été assurée avec un fluide dont au moins une caractéristique respecte une contrainte prédéterminée.

On peut encore citer le document FR-2 735 381 qui propose une installation de fourniture d'un gaz incorporant un dispositif de détection d'impuretés, permettant sous l'effet d'un signal fourni par le poste d'analyse de dériver le flux de gaz analysé avant que l'impureté ne puisse atteindre la partie aval de la ligne et donc l'application finale.

Ainsi, de l'avis de la Demanderesse aucun de ces documents ne fournit de solution pleinement satisfaisante permettant de garantir au moins une caractéristique d'un fluide utilisé pour la production de produits alimentaires et donc de répondre aux exigences croissantes de l'industrie alimentaire en matière de sécurité et de qualité.

Un but de l'invention est de résoudre ce problème.

II faut en effet signaler que si le producteur et/ou le fournisseur de gaz garantissent couramment aux utilisateurs la qualité des gaz livrés, en revanche aucune garantie ou aucun contrôle systématique de la qualité des gaz au niveau chimique, physique et/ou microbiologique n'est assuré au point d'utilisation. L'impact du réseau sur la qualité du gaz n'est pas suivi, de même le maintien dans la durée de la qualité des gaz au point d'utilisation n'est pas contrôlé.

La mise en place généralisée des méthodes HACCP dans les entreprises alimentaires conduit les utilisateurs à mettre en place des points critiques de contrôle où des risques (microbiologique, physique, chimique) peuvent apparaître.

La qualité des gaz au point d'utilisation est donc un point critique à contrôler dans le cadre de cette démarche afin de s'assurer que le gaz en contact avec les aliments n'est pas source de pollution.

Selon la présente invention on se propose de formuler une approche globale pour maîtriser et/ou garantir la qualité des gaz ou du mélange gazeux de la production au point d'utilisation au niveau microbiologique, physique et chimique.

On inclut préférentiellement la mise en place de mesures permettant d'éliminer des contaminants chimiques, physiques et/ou microbiologiques au point d'utilisation, la mise en place de système de contrôle permettant de vérifier la qualité des gaz ou du mélange gazeux jusqu'au point d'utilisation, la mise en place d'un système de d'enregistrement en continu permettant d'archiver les informations (mesures, entretiens, défaillances) qui se produisent sur la chaîne de distribution des gaz et la mise en place d'un système de traçabilité permettant de relier la fourniture des gaz aux lots de production du client.

A cet effet, l'invention a pour objet un procédé pour garantir au moins une caractéristique d'un fluide utilisé pour la production de produits alimentaires comprenant les étapes de :
- fournir un réseau de canalisation et de distribution du fluide associé à des moyens de production des produits pour utiliser le fluide pour la production des produits,
- mesurer une valeur de la caractéristique,
- comparer la valeur mesurée à une valeur seuil prédéterminée,
- exécuter une action si la valeur mesurée franchit la valeur seuil,
et se caractérisant par la mise en oeuvre des étapes suivantes :
- associer la valeur mesurée à une première information d'identification d'au moins un produit, et
- stocker la valeur et l'information associées.

Selon des modes particuliers de réalisation, le procédé peut comprendre l'une ou plusieurs des caractéristiques suivantes, prise(s) isolément ou selon toutes les combinaisons techniquement possibles :
- l'action comprend une étape d'émission d'un signal d'alarme.
- l'action comprend au moins une étape exercée sur au moins une partie du réseau.
- l'action comprend une étape de nettoyage et/ou de stérilisation d'au moins une partie du réseau.
- l'action comprend une étape d'enlèvement d'une partie du réseau puis de remplacement de la partie enlevée par une nouvelle partie.
- l'action comprend une étape de substitution d'une partie du réseau à une autre partie du réseau.
- ladite partie du réseau est une source du fluide.
- l'action comprend une étape d'arrêt de la distribution du gaz.
- le réseau comprend un dispositif de purification du fluide, en ladite caractéristique est une caractéristique relative à la pureté, et l'on mesure la valeur de la caractéristique en aval du dispositif de purification.
- la caractéristique est relative à la teneur en impureté physique, chimique ou microbiologique.
- la première information d'identification est une information intermédiaire et temporelle d'identification.
- la première information d'identification est fournie par une première horloge, et il comprend en outre une étape d'associer une deuxième information intermédiaire et temporelle d'identification fournie par une deuxième horloge à une troisième information finale d'identification.
- la première information d'identification est une information finale d'identification.

L'invention sera mieux comprise à la lecture de la description qui va suivre, donnée uniquement à titre d'exemple, et faite en se référant aux dessins annexés, sur lesquels :
- La figure 1 est une vue schématique d'une installation selon un premier mode de réalisation de l'invention,
- La figure 2 est une vue schématique d'une variante de l'installation de la figure 1,
- La figure 3 est une vue schématique d'une installation selon un deuxième mode de réalisation de l'invention, et
- La figure 4 est une vue schématique d'une installation selon un troisième mode de réalisation.

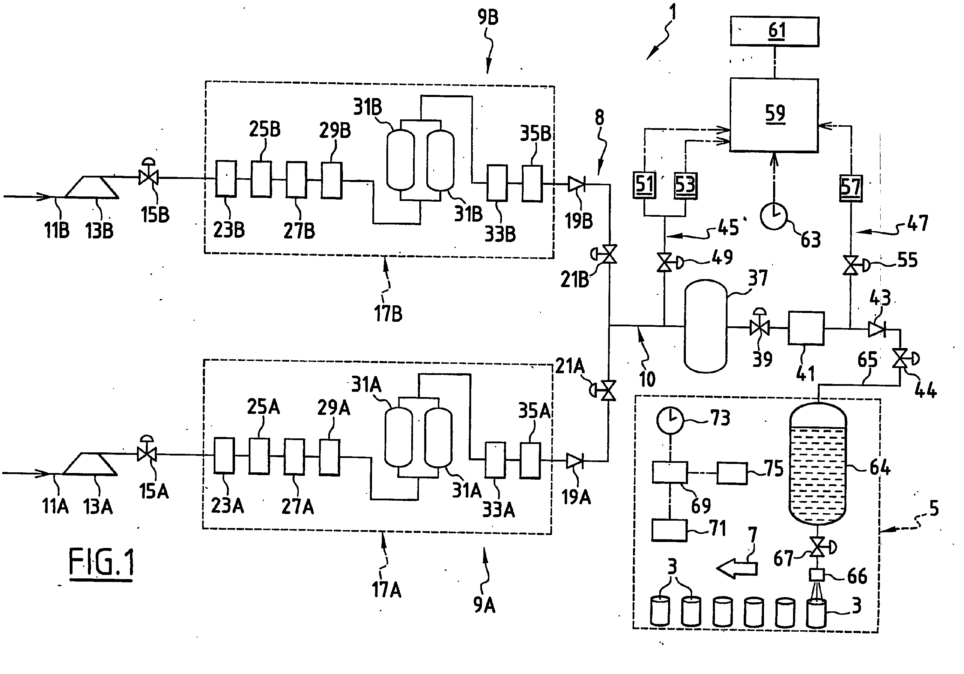

La figure 1 illustre schématiquement une installation 1 de production de produits 3 en utilisant de l'air comprimé comme auxiliaire technologique. La production des produits 3 est plus spécifiquement mise en oeuvre par des moyens 5 de production qui assurent, par exemple, une production en chaîne comme illustré par la flèche 7 sur la figure 1.

L'installation 1 comprend un réseau 8 de canalisation et de distribution d'air comprimé. Ce réseau 8 comprend deux lignes amonts 9A et 9B de canalisation d'air raccordées à une ligne aval 10 de canalisation d'air, elle-même raccordée aux moyens 5 de production.

Les structures des lignes 9A et 9B étant analogues, les mêmes références numériques seront utilisées, suivies soit du suffixe A pour la ligne 9A, soit du suffixe B pour la ligne 9B. Pour la même raison, seule la structure et le fonctionnement de la ligne 9A seront décrits en détail par la suite. La ligne 9A comprend successivement de l'amont vers l'aval :
- une conduite 11 A de mise en communication avec l'atmosphère ambiante qui forme source d'air,
- un compresseur d'air 13A,
- une vanne 15A,
- une unité 17A de purification,
- un clapet anti- retour 19A, et
- une vanne 21A.

L'unité de purification 17A comprend successivement de l'amont vers l'aval :
- un filtre cyclonique 23A,
- un préfiltre 25A d'élimination des particules de taille supérieure à 25µm,
- un filtre submicronique 27A d'élimination des particules de dimensions supérieures à 0,1 µm, par exemple un filtre à coalescence,
- un filtre submicronique 29A d'élimination des particules de dimensions supérieures à 0,01 µm, par exemple un filtre à coalescence,
- deux dispositifs de dessiccation 31A disposés en parallèle et comprenant chacun un récipient rempli d'un adsorbeur tel que de l'alumine,
- un filtre 33A à charbon actif, et
- un filtre 35A à poussières, par exemple un filtre en matériau fritté.

On notera que les différents éléments de la ligne amont 9A sont des éléments classiques. La ligne aval 10 comprend successivement de l'amont vers l'aval :
- une capacité tampon 37,
- une vanne 39,
- un filtre bactériologique 41, par exemple un filtre en membrane plissée hydrophobe,
- un clapet anti-retour 43, et
- une vanne 44.

Ces différents éléments sont également des éléments classiques.

La ligne aval 10 est raccordée en amont de la capacité 37 à une première ligne de dérivation 45 et, entre le filtre bactériologique 41 et le clapet anti-retour 43, à une deuxième ligne de dérivation 47. La ligne de dérivation 45 est munie d'une vanne 49 et est raccordée en parallèle à un capteur 51 de mesure de la teneur en eau ou hygromètre, et à un capteur 53 de mesure de la teneur en CO et en CO₂. Ces capteurs sont également des éléments classiques.

La seconde ligne de dérivation 47 est munie d'une vanne 55 et est raccordée à un capteur 57 de mesure d'une information relative à la pureté microbiologique, par exemple un capteur capable de déterminer la teneur en particules biotiques comme décrit dans WO-98/48 259.

L'installation 1 comprend en outre une unité électronique de traitement d'informations 59 et, raccordées à cette unité 59, une mémoire 61 et une horloge 63. L'unité 59 comprend notamment un microprocesseur convenablement programmé pour assurer les opérations ultérieurement décrites. Par ailleurs, les capteurs 51, 53 et 57 sont raccordés à l'unité 59 pour lui fournir des informations relatives aux caractéristiques ou grandeurs qu'ils mesurent.

Les moyens 5 de production décrits ci-après seront, à titre d'exemple, des moyens de production de récipients 3 contenant du lait. Il pourrait également s'agir de récipients contenant une crème dessert. La description de ces moyens 5 se bornera aux éléments nécessaires à la description de l'invention et sera donc très schématique, le reste de ces moyens 5 étant par ailleurs classique.

Les moyens 5 comprennent une cuve 64 contenant du lait. Le sommet de cette cuve 64 est raccordé au tronçon aval 65 de la ligne 10. La cuve 64 alimente par son fond des moyens 66 de remplissage des récipients 3. Une vanne 67 est disposée entre le fond de la cuve 64 et les moyens 66 de remplissage.

Les moyens 5 de production comprennent en outre une unité électronique de traitement d'informations 69 et, reliés à celle-ci, des moyens 71 pour munir les produits 3 d'une information finale d'identification, par exemple un numéro de lot, une horloge 73 synchronisée avec l'horloge 63 et une mémoire 75. L'unité 69 comprend notamment un microprocesseur convenablement programmé pour assurer les opérations décrites ultérieurement.

Le fonctionnement de l'installation 1 est le suivant. Les vannes 15A et 21 A sont ouvertes, tandis que les vannes 15B et 21B sont fermées.

De l'air de l'atmosphère extérieur est canalisé par la conduite 11A, comprimé par le compresseur 13A puis subit une pré-dessiccation dans le filtre 23A permettant d'éliminer environ 96% en masse de l'eau contenue dans l'air. Ensuite, les filtres 25A, 27A et 29A éliminent la majeure partie des hydrocarbures que l'air peut contenir, et notamment les huiles. Typiquement, la teneur en huile de l'air en sortie du filtre 29A est inférieure à 0,01 ppm.

L'air traverse ensuite un des dispositifs 31 A où sa dessication est poursuivie par adsorption. L'autre dispositif 31 A est alors en phase de régénération par élution comme cela est classique, par exemple à l'aide d'un débit d'air sec prélevé en sortie de la capacité tampon 37. Typiquement, le point de rosée en sortie du dispositif 31 A utilisé est supérieur ou égal à -40°C.

L'air dessiqué traverse ensuite le filtre 33A où les dernières traces et les odeurs d'huile sont sensiblement éliminées (teneur résiduelle voisine de 0,003 ppm), puis le filtre 35A qui élimine les poussières contenues dans l'air.

En sortie de l'unité 17A, l'air contient par m³, moins de 3520 particules de dimensions supérieures ou égales à 0,5 µm (Classe ISO 5 selon les classes définies par ISO 14644-1). L'hygrométrie de l'air est alors inférieure à 0,05% et sa teneur en hydrocarbures inférieure à 100µl/l (0,09 mg/m³).

L'air ainsi comprimé, dessiqué, dépoussiéré et déshuilé est ensuite envoyé vers la capacité tampon 37.

La vanne 39 est ouverte pour soutirer de l'air de la capacité 37. Les micro-organismes présents dans cet air sont éliminés par le filtre 41. La vanne 44 étant ouverte, l'air ainsi comprimé et purifié est distribué au sommet de la cuve 64 par le tronçon aval 65 de la ligne 10. L'air comprimé pousse alors le lait vers le fond de la cuve 64, en favorisant sa sortie de la cuve 64 et donc le remplissage des récipients 3.

La production des produits 3 est ainsi assurée grâce à de l'air comprimé et purifié fourni uniquement par la ligne 9A et la ligne 10, la ligne 9B n'étant pas utilisée.

Pendant cette production, les vannes 49 et 55 des lignes de dérivation 45 et 47 sont ouvertes pour permettre aux capteurs 51, 53 et 57 d'acquérir et de fournir à l'unité 59 :
- une mesure de l'hygrométrie de l'air utilisé pour la production des produits 3,
- une mesure de la teneur en CO et CO₂ de l'air utilisé, cette teneur étant un traceur d'une possible dérive de la teneur en huile de l'air utilisé pour la production des produits 3, et
- une mesure de la teneur en particules biotiques, cette teneur étant représentative de la teneur en impuretés microbiologiques de l'air utilisé pour la production des produits 3.

Ces différentes informations transmises à l'unité 59 y sont associées à une information temporelle fournie par l'horloge 63. Ces informations associées sont alors stockées dans la mémoire 61. Ainsi, ces informations stockées permettent de connaître pour un instant donné, ou pour une période de temps donnée, la pureté, en terme d'humidité, de teneur CO/CO₂ et de teneur en impuretés microbiologiques, de l'air utilisé pour la production des produits 3.

De manière analogue, l'unité électronique de traitement d'informations 69 associe les informations finales d'identification des produits 3 fournies par les moyens 71 à des informations temporelles fournies par l'horloge 73 et les stocke dans la mémoire 75. Ainsi, pour des produits 3 donnés, il est possible, de connaître l'instant, ou la période de temps, où ils ont été produits.

L'installation 1 de la figure 1 enregistrant les informations de pureté de l'air utilisé pour la production des produits 3, il est possible de contrôler que cette production a été effectuée dans des conditions de sécurité et de qualité satisfaisantes.

Par ailleurs, l'utilisateur de l'installation 1 est capable de prouver que des produits 3 donnés ont été produits en utilisant de l'air de pureté satisfaisante.

En effet, il est possible, pour des produits 3 donnés, de connaître à quel instant, ou pendant quelle période de temps, ils ont été produits, grâce aux informations stockées dans la mémoire 75. Cette information temporelle permet alors, grâce aux informations stockées dans la mémoire 61, de connaître les informations de pureté de l'air utilisé à cet instant, ou pendant cette période de temps. On notera que les informations temporelles fournies par les horloges 63 et 73 constituent des informations intermédiaires d'identification. En outre, on notera qu'une seule et même horloge peut être utilisée à la place de ces deux horloges pour fournir aux unités 59 et 69 les mêmes informations intermédiaires d'identification.

L'installation 1 permet donc de mettre en place des démarches de traçabilité et de qualité permettant de satisfaire aux exigences accrues de sécurité dans le domaine alimentaire.

L'unité électronique de traitement d'informations 59 peut en outre être adaptée pour commander la fermeture des vannes 15A et 21A d'une part, et l'ouverture des vannes 15B et 21B d'autre part, pour que la compression de l'air et sa purification, en amont de la capacité 37, soient assurées par la ligne 9B plutôt que par la ligne 9A. Cette commande peut être assurée dès que l'unité 59 détermine par comparaison que la teneur en eau ou en huile a dépassé une valeur seuil prédéterminée respective stockée dans la mémoire 61. Ainsi, l'unité 59 peut commander le raccordement sélectif des lignes 9A et 9B à la ligne 10 afin de garantir la qualité de l'air utilisé pour la production des produits 3.

De manière plus générale, les informations de pureté reçues par l'unité 59 peuvent être utilisées pour commander diverses actions sur les lignes 9A, 9B et 10 afin de corriger des défauts de pureté constatés.

On notera également que l'installation 1 peut ne comprendre qu'une seule ligne amont 9 (une autre variante peut être l'utilisation d'un seul moyen de mise en communication avec une source de fluide à savoir utilisation d'un seul compresseur relié aux deux lignes de purification 9 A et 9B).

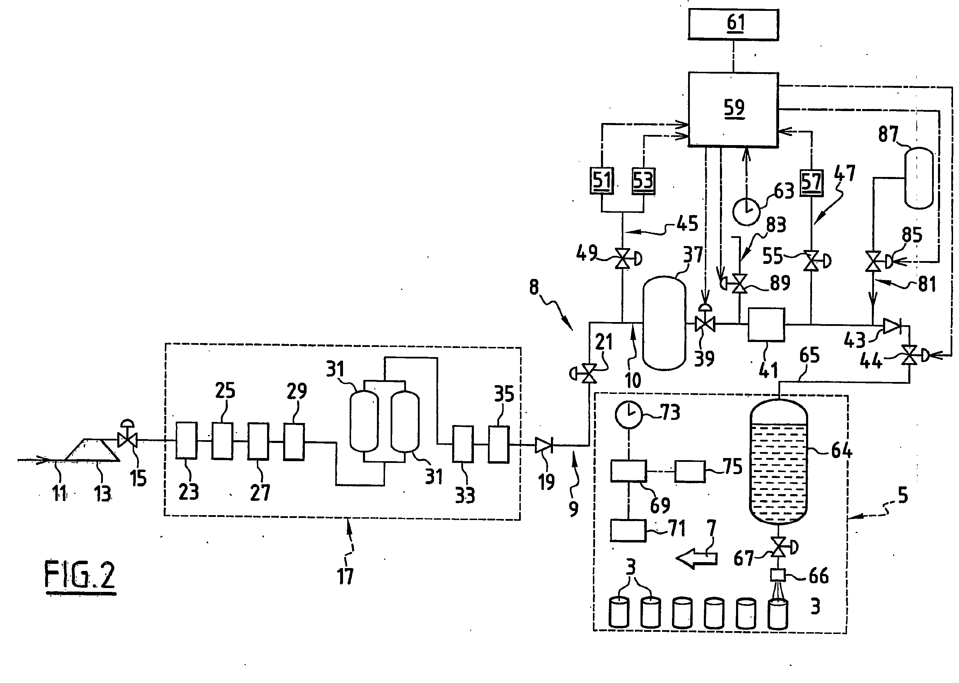

Ainsi, la figure 2 illustre une variante de l'installation 1 qui se distingue de celle décrite ci-dessus par le fait que le réseau 8 ne comprend qu'une ligne amont 9. La ligne aval 10 est munie d'une troisième ligne de dérivation 81 située entre la ligne de dérivation 47 et le clapet anti-retour 43, et d'une quatrième ligne de dérivation 83 située entre la vanne 39 et le filtre 41.

La ligne de dérivation 81 est munie d'une vanne 85 et est reliée à une source 87 d'un fluide de nettoyage et/ou de stérilisation, par exemple du STEROXAL (marque déposée) commercialisé par la société L'AIR LIQUIDE ou une source de vapeur.

La ligne de dérivation 83 est mise à l'air à son extrémité opposée à celle la reliant à la ligne aval 10. On notera cependant qu'elle pourrait être raccordée à la ligne 81 d'une manière permettant de recycler le fluide de nettoyage et/ou de stérilisation utilisé.

Lorsque l'unité 59 détermine par comparaison que l'information de teneur en impuretés microbiologiques fournie par le capteur 57 est supérieure à une valeur seuil prédéterminée stockée dans le mémoire 61, l'unité 59 commande alors la fermeture des vannes 39 et 44 et l'ouverture des vannes 85 et 89.

Le fluide du réservoir 87 traverse alors le filtre 41 en le stérilisant puis est mis à l'air par la ligne 83. Cette opération de nettoyage et/ou de stérilisation se poursuit pendant une durée prédéterminée puis l'unité 59 commande la fermeture des vannes 85 et 89 et l'ouverture des vannes 39 et 44.

Ainsi, l'unité 59 est adaptée pour assurer un nettoyage et/ou une stérilisation de la ligne 10 en cas de besoin, ce qui permet de garantir de manière encore plus importante la qualité de l'air utilisé pour la production des produits 3.

On notera que le capteur 57 peut être remplacé par un dispositif de prélèvement ponctuel d'échantillons de gaz qui peuvent être analysés en terme de contamination microbiologique par un laboratoire situé sur un site distinct de celui de l'installation 1. Les informations de pureté microbiologique fournies par le laboratoire sont associées, avec les informations de pureté fournies par les capteurs 51 et 53, à l'information temporelle fournie par l'horloge 63. Les informations ainsi associées sont stockées dans la mémoire 61.

Les principes de purification, acquisition d'informations relatives à la pureté, et enregistrement de ces informations peuvent être appliqués à tous types de gaz ou même de fluides utilisés dans la production de produits. En particulier, le fluide utilisé pour la production des produits peut être distribué sous forme liquide.

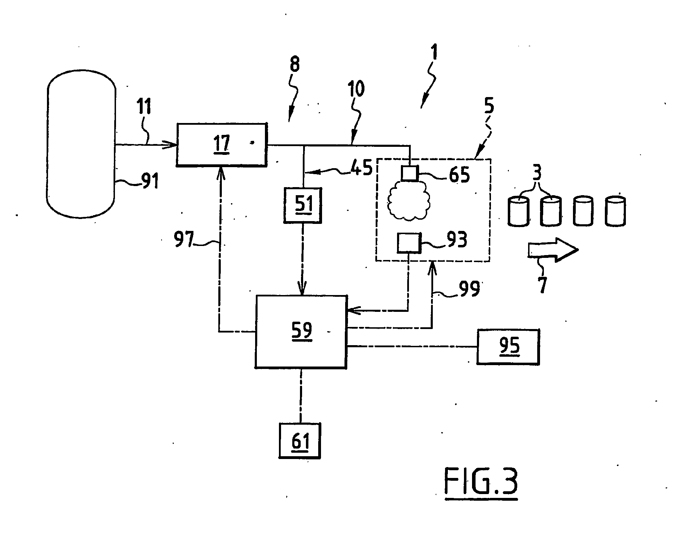

La figure 3 illustre ainsi une forme générale de l'invention dans laquelle le réseau 8 comprend d'amont en aval un réservoir 91 de stockage, par exemple sous forme liquide, d'un fluide à distribuer, une conduite 11, une unité de purification 17, une ligne 10 et des moyens 65 de distribution, par exemple sous forme gazeuse comme dans les exemples des figures 1 et 2, du fluide purifié. Ces moyens 65 sont associés à des moyens 5 de production de produits 3.

Un capteur 51 permet, grâce à une ligne de dérivation 45, de mesurer une caractéristique relative à la pureté du fluide en aval de l'unité 17 de purification. Ce capteur 51 transmet cette information à l'unité électronique de traitement d'informations 59 qui reçoit également des informations finales d'identification des produits 3 fournies par des moyens 93 d'identification. Les informations de pureté fournies par le capteur 51 et les informations d'identification des produits fournies par les moyens 93 sont associées par l'unité 59 puis stockées dans la mémoire 61 afin de permettre de connaître, pour des produits 3 donnés, quelle était l'information de pureté acquise par le capteur 51 pour le fluide utilisé pour la production de ces produits 3.

Ces informations associées par l'unité 59 peuvent également être envoyées via un dispositif de communication à distance 95, tel qu'un modem, à une installation de surveillance éloignée du site de l'installation 1.

Comme illustré par les flèches 97 et 99, l'unité 59 peut également être adaptée pour agir sur l'unité de purification 17 ou sur les moyens 5 de production des produits 3 en fonction des informations reçues du capteur 51.

Il pourra s'agir par exemple de déclencher un nettoyage et/ou une stérilisation des lignes 10 et 11 et/ou de l'unité 17 lorsque l'unité 59 détermine par comparaison que la valeur mesurée par le capteur 51 franchit une valeur seuil prédéterminée et stockée dans la mémoire 61.

De manière générale, l'unité 17 peut être une unité adaptée pour éliminer les impuretés physiques, telles que des poussières, des impuretés chimiques, telles que de l'eau, ou des impuretés microbiologiques telles que des bactéries.

Dans l'exemple de la figure 3, l'association des informations fournies par le capteur 51 aux informations finales d'identification des produits 3 permet de corréler les informations de pureté aux produits 3 sans utiliser d'information intermédiaire d'identification, telle qu'une information temporelle fournie par une horloge.

On notera que la corrélation des informations fournies par le capteur 51 aux produits 3, y compris via des informations temporelles, n'est pas indispensable, le seul stockage des informations fournies par le capteur 51 dans la mémoire 61 permettant de prouver que des conditions de sécurité et de qualité ont bien été remplies lors de la production d'au moins certains produits 3.

On notera également que le ou les unités ou dispositifs de purification seront en général disposés en aval de tronçons critiques du réseau 8. Par ailleurs, il est préférable de disposer le ou les capteurs de mesure de pureté au plus près des moyens 65 de distribution du fluide.

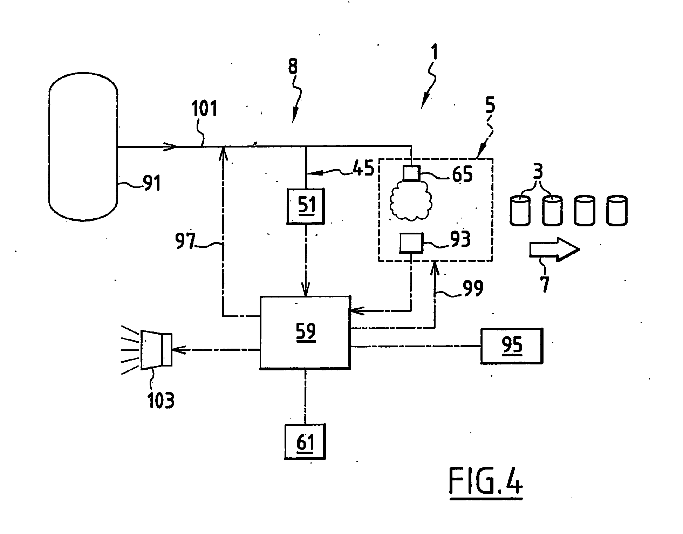

La figure 4 illustre de manière générale une installation 1 dédiée à la production de produits alimentaires 3. Ainsi, le réseau 8 est réalisé à partir d'éléments spécifiquement adaptés à l'industrie alimentaire et permettant en particulier de limiter les risques de contamination chimique, physique et microbiologique. En outre, l'installation se distingue par ce qui suit de celle de la figure 3.

Le réseau 8, dont la structure n'a pas été détaillée sur la figure 4, peut comprendre tous types d'éléments, et notamment des unités ou dispositifs de purification, bien que cela ne soit pas indispensable contrairement au cas de la figure 3.

Le capteur 51 mesure une valeur d'une caractéristique du fluide qui peut, sans que cela ne soit nécessaire, être relative à sa pureté. Ainsi, cette caractéristique peut être une teneur en impureté physique, chimique ou biologique, mais également la température, la pression... L'unité électronique de traitement d'informations 59 assure :
- la comparaison de la valeur mesurée par le capteur 51 avec une valeur seuil prédéterminée stockée dans la mémoire 61 qui correspond à une valeur que l'on souhaite garantir pour la caractéristique,
- l'association des valeurs fournies par le capteur 51 avec les informations finales d'identification fournies par les moyens 93 d'identification afin de corréler chaque valeur mesurée au(x) produit(s) 3 dont la production a utilisé le fluide avec la caractéristique à la valeur mesurée, et
- le stockage des valeurs et des informations ainsi associées dans la mémoire 61.

En cas de franchissement de la valeur seuil prédéterminée par la valeur mesurée, l'unité électronique 59 est en outre adaptée pour déclencher l'exécution d'actions.

Certaines de ces actions peuvent être exercées sur au moins une partie 101 du réseau 8 de canalisation et de distribution, comme illustré par la flèche 97. Il peut s'agir par exemple de l'enlèvement de la partie 101 et son remplacement par une nouvelle partie 101.

Il peut également s'agir de la substitution d'une partie du réseau 8 à une autre pour assurer la canalisation et la distribution du fluide, comme cela a été décrit en regard de la figure 1 pour la substitution de la ligne amont 9B à la ligne amont 9A. Dans ce cas, l'exécution de l'action est assurée par l'unité 59.

Il peut également s'agir de l'arrêt de la distribution du fluide par fermeture d'une vanne du réseau 8 par l'unité 59 ou de l'enlèvement de la source 91 et de son remplacement par une nouvelle source 91.

Des actions peuvent également être exercées par l'unité 59 sur les moyens 5 de production des produits 3, comme illustré par la flèche 99, il peut alors s'agir par exemple de la mise à l'arrêt de ces moyens 5 de production après mise en sécurité de la ligne de distribution de fluide (arrêt de l'approvisionnement en fluide).

En outre, une action peut également être l'émission d'un signal d'alarme par un dispositif 103.

Il peut s'agir comme schématisé par la figure 4 d'un signal sonore émis par un haut-parleur, mais également d'un signal optique, émis par exemple par un écran de surveillance.

L'installation 1 permet donc l'exécution de mesures correctives suite à la détection du non-respect des contraintes imposées à la caractéristique du fluide.

En outre, l'association des valeurs mesurées aux informations d'identification des produits et le stockage ultérieur dans la mémoire 61 permet de contrôler a posteriori quelle était la valeur de la caractéristique du fluide utilisé pour la production de certains produits 3.

Ainsi, l'installation 1 permet de mettre en place des démarches de traçabilité, et contribue encore plus à la garantie que la caractéristique du fluide utilisé respecte bien certaines contraintes prédéterminées. On notera que la valeur seuil peut être une valeur maximale à respecter ou une valeur minimale à respecter. Ainsi, l'unité électronique 59 peut déclencher l'exécution des différentes actions lors d'un dépassement de la valeur seuil ou lors d'une diminution sous la valeur seuil.

On notera enfin que les valeurs mesurées peuvent être associées non pas à des informations finales d'identification mais à des informations intermédiaires d'identification, par exemple des informations temporelles fournies par une horloge comme décrit en regard des figures 1 et 2.

## Revendications

1. Procédé pour garantir au moins une caractéristique d'un fluide utilisé pour la production de produits alimentaires (3), comprenant les étapes de :
- fournir un réseau (8) de canalisation et de distribution du fluide associé à des moyens (5) de production des produits (3) pour utiliser le fluide pour la production des produits (3),
- mesurer une valeur de la caractéristique,
- comparer la valeur mesurée à une valeur seuil prédéterminée,
- exécuter une action si la valeur mesurée franchit la valeur seuil,
et **se caractérisant par** la mise en oeuvre des étapes suivantes :
i) associer la valeur mesurée à une première information d'identification d'au moins un produit (3), et j) stocker la valeur et l'information associées.

2. Procédé selon la revendication 1, **caractérisé en ce que** l'action comprend une étape d'émission d'un signal d'alarme.

3. Procédé selon la revendication 1 ou 2, **caractérisé en ce que** l'action comprend au moins une étape exercée sur au moins une partie (9A, 9B; 10; 91; 101) du réseau (8).

4. Procédé selon la revendication 3, **caractérisé en ce que** l'action comprend une étape de nettoyage et/ou de stérilisation d'au moins une partie (10; 101) du réseau (8).

5. Procédé selon la revendication 3 ou 4, **caractérisé en ce que** l'action comprend une étape d'enlèvement d'une partie (91;101) du réseau (8) puis de remplacement de la partie enlevée par une nouvelle partie.

6. Procédé selon l'une des revendications 3 à 5, **caractérisé en ce que** l'action comprend une étape de substitution d'une partie (9B) du réseau (8) à une autre partie (9A) du réseau (8).

7. Procédé selon la revendication 5 ou 6, **caractérisé en ce que** ladite partie du réseau (8) est une source du fluide (91).

8. Procédé selon l'une des revendications 3 à 7, **caractérisé en ce que** l'action comprend une étape d'arrêt de la distribution du gaz.

9. Procédé selon l'une des revendications précédentes, **caractérisé en ce que** le-réseau (8)--comprend un dispositif (17) de purification du fluide, **en ce que** ladite caractéristique est une caractéristique relative à la pureté, et **en ce que** l'on mesure la valeur de la caractéristique en aval du dispositif de purification.

10. Procédé selon la revendication 9, **caractérisé en ce que** la caractéristique est relative à la teneur en impureté physique, chimique ou microbiologique.

11. Procédé selon l'une des revendications précédentes, **caractérisé en ce que** la première information d'identification est une information intermédiaire et temporelle d'identification.

12. Procédé selon la revendication 11, **caractérisé en ce que** la première information d'identification est fournie par une première horloge, et **en ce qu'**il comprend en outre une étape d'associer une deuxième information intermédiaire et temporelle d'identification fournie par une deuxième horloge à une troisième information finale d'identification.

13. Procédé selon l'une des revendications 1 à 10, **caractérisé en ce que** la première information d'identification (3) est une information finale d'identification.

## Claims

1. Method for guaranteeing at least one characteristic of a fluid used for producing food products (3), comprising the steps of:
- providing a system (8) for piping and delivering the fluid, combined with means (5) for producing the products (3), in order to use the fluid for producing the products (3);
- measuring a value of the characteristic;
- comparing the measured value with a predetermined threshold value;
- executing an action if the measured value departs from the threshold value;
and **characterized by** the use of the following steps:
i) combining the measured value with first information for identifying at least one product (3); and
ii) storing the associated value and the associated information.

2. Method according to Claim 1, **characterized in that** the action comprises a step of issuing an alarm signal.

3. Method according to Claim 1 or 2, **characterized in that** the action comprises at least one step carried out on at least one part (9A, 9B; 10; 91; 101) of the system (8).

4. Method according to Claim 3, **characterized in that** the action comprises a step of cleaning and/or sterilizing at least one part (10; 101) of the system (8).

5. Method according to Claim 3 or 4, **characterized in that** the action comprises a step of removing one part (91; 101) of the system (8) and then replacing the removed part with a new part.

6. Method according to one of Claims 3 to 5, **characterized in that** the action comprises a step of substituting one part (9B) of the system (8) with another part (9A) of the system (8).

7. Method according to Claim 5 or 6, **characterized in that** said part of the system (8) is a source of the fluid (91).

8. Method according to one of Claims 3 to 7, **characterized in that** the action comprises a step of stopping the delivery of the gas.

9. Method according to one of the preceding Claims, **characterized in that** the system (8) comprises a device (17) for purifying the fluid, **in that** said characteristic is a characteristic relating to the purity and **in that** the value of the characteristic is measured downstream of the purification device.

10. Method according to Claim 9, **characterized in that** the feature relates to the content of a physical, chemical or microbiological impurity.

11. Method according to one of the preceding claims, **characterized in that** the first identification information is intermediate and temporal identification information.

12. Method according to Claim 11, **characterized in that** the first identification information is delivered by a first clock and **in that** it furthermore includes a step of associating second, intermediate and temporal, identification information delivered by a second clock with third, final identification information.

13. Method according to one of Claims 1 to 10, **characterized in that** the first identification information (3) is a final identification information.

## Patentansprüche

1. Verfahren zur Gewährleistung mindestens eines Merkmals eines Fluids, das für die Herstellung von Nahrungsmitteln (3) verwendet wird, umfassend die folgenden Schritte:
- Bereitstellung eines Netzes (8) zur Kanalisierung und Verteilung des Fluids in Verbindung mit Mitteln (5) zur Herstellung der Produkte (3), um das Fluid für die Herstellung der Produkte (3) zu verwenden,
- Messen eines Wertes des Merkmals,
- Vergleichen des gemessenen Wertes mit einem vorbestimmten Grenzwert,
- Ausführen einer Handlung, wenn der gemessene Wert den Grenzwert überschreitet,
und **gekennzeichnet durch** den Einsatz der folgenden Schritte:
i) Verbinden des gemessenen Wertes mit einer Information zur Identifikation mindestens eines Produktes (3), und
ii) Speichern des Wertes und der zugehörigen Information.

2. Verfahren nach Anspruch 1, **dadurch gekennzeichnet, dass** die Handlung einen Schritt des Sendens eines Warnsignals umfasst.

3. Verfahren nach Anspruch 1 oder 2, **dadurch gekennzeichnet, dass** die Handlung mindestens einen Schritt umfasst, der an mindestens einem Teil (9A, 9B; 10; 91; 101) des Netzes (8) durchgeführt wird.

4. Verfahren nach Anspruch 3, **dadurch gekennzeichnet, dass** die Handlung einen Schritt des Reinigens und/oder Sterilisierens mindestens eines Teils (10; 101) des Netzes (8) umfasst.

5. Verfahren nach Anspruch 3 oder 4, **dadurch gekennzeichnet, dass** die Handlung einen Schritt der Entnahme eines Teils (91; 101) des Netzes (8) und dann des Ersatzes des entnommenen Teils durch einen neuen Teil umfasst.

6. Verfahren nach einem der Ansprüche 3 bis 5,
**dadurch gekennzeichnet, dass** die Handlung einen Schritt der Substitution eines Teils (9B) des Netzes (8) für einen anderen Teil (9A) des Netzes (8) umfasst.

7. Verfahren nach Anspruch 5 oder 6, **dadurch gekennzeichnet, dass** der Teil des Netzes (8) eine Fluidquelle (91) ist.

8. Verfahren nach einem der Ansprüche 3 bis 7, **dadurch gekennzeichnet, dass** die Handlung einen Schritt der Einstellung der Verteilung des Gases umfasst.

9. Verfahren nach einem der vorhergehenden Ansprüche, **dadurch gekennzeichnet, dass** das Netz (8) eine Vorrichtung (17) zur Reinigung des Fluids umfasst, dass dieses Merkmal ein die Reinheit betreffendes Merkmal ist, und dass der Wert des Merkmals stromabwärts zur der Reinigungsvorrichtung gemessen wird.

10. Verfahren nach Anspruch 9, **dadurch gekennzeichnet, dass** sich das Merkmal auf den Gehalt an physikalischer, chemischer oder mikrobiologischer Unreinheit bezieht.

11. Verfahren nach einem der vorhergehenden Ansprüche, **dadurch gekennzeichnet, dass** die erste Identifikationsinformation eine Zwischen- und Zeitinformation der Identifikation ist.

12. Verfahren nach Anspruch 11, **dadurch gekennzeichnet, dass** die erste Identifikationsinformation von einer ersten Schaltuhr geliefert wird, und dass es ferner einen Schritt der Verbindung einer zweiten Zwischen- und Zeitinformation der Identifikation, die von einer zweiten Schaltuhr geliefert wird, mit einer dritten endgültigen Identifikationsinformation umfasst.

13. Verfahren nach einem der Ansprüche 1 bis 10, **dadurch gekennzeichnet, dass** die erste Identifikationsinformation (3) eine endgültige Identifikationsinformation ist.
